(19) Europäisches Patentamt
European Patent Office
Office européen des brevets

(11) Publication number: **0 206 411**
**A2**

# (12) EUROPEAN PATENT APPLICATION

(21) Application number: **86201042.8**

(22) Date of filing: **16.06.86**

(51) Int. Cl.⁴: **C 12 Q 1/68**, B 01 D 15/08
// G01N33/543

(30) Priority: **17.06.85 US 745547**

(43) Date of publication of application: **30.12.86**
**Bulletin 86/52**

(84) Designated Contracting States: **BE IT LU NL SE**

(71) Applicant: **THE BOARD OF TRUSTEES OF THE UNIVERSITY OF ILLINOIS, 349 Administration Building University of Illinois 506 South Wright Street, Urbane Illinois 61801 (US)**

(72) Inventor: **Jagadeeswaran, Pudur, 901 S. Ashland, Chicago Illinois 60607 (US)**

(74) Representative: **Kooy, Leendert Willem et al, OCTROOIBUREAU VRIESENDORP & GAADE P.O. Box 266, NL-2501 AW The Hague (NL)**

(54) **Reverse phase chromatography in oligonucleotide sequencing.**

(57) The present invention discloses a new and novel method for the sequencing of nucleic acid bases in oligonucleotides such as DNA.

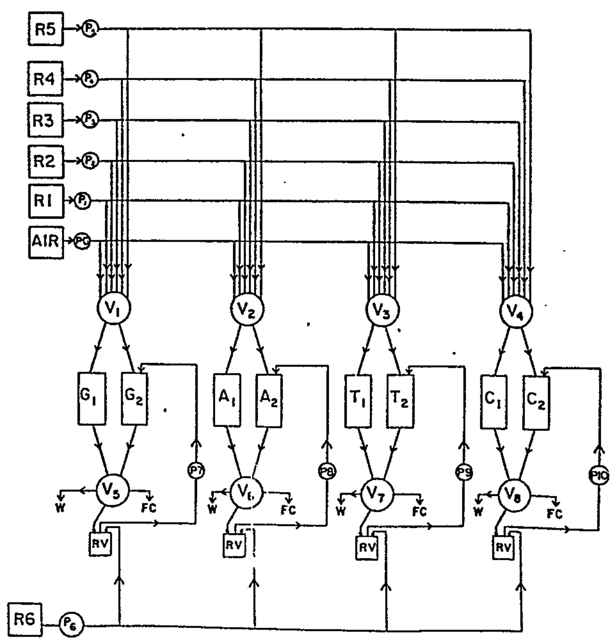


EP 0 206 411 A2

REVERSE PHASE CHROMATOGRAPHY IN
OLIGONUCLEOTIDE SEQUENCING

The sequencing of oligonucleotides, particularly deoxyribonucleic acid or DNA, is a valuable tool in the understanding of the structure and function of the DNA molecule, the fundamental substance of genes. Watson and Crick were the first to identify the structure of DNA as being two complementary oligonucleotide strands linked together by hydrogen bonding in the form of a "double helix" of four independent nucleosides linked together by phosphodiester bridges.

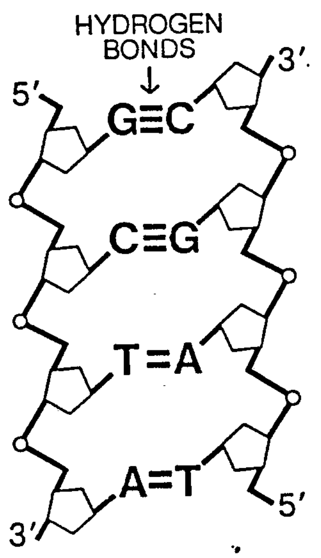


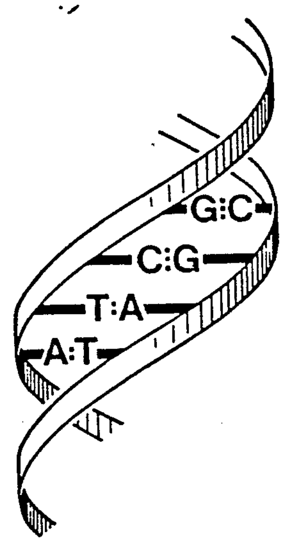


Each nucleoside of the DNA molecule consists of a deoxyribose sugar molecule, linked together at the 3' and 5' positions by the phosphodiester bridge, and one of four possible nucleic bases - adenine (A), guanine (G), thymine (T), or cytosine (C) - carried at the 1' position.

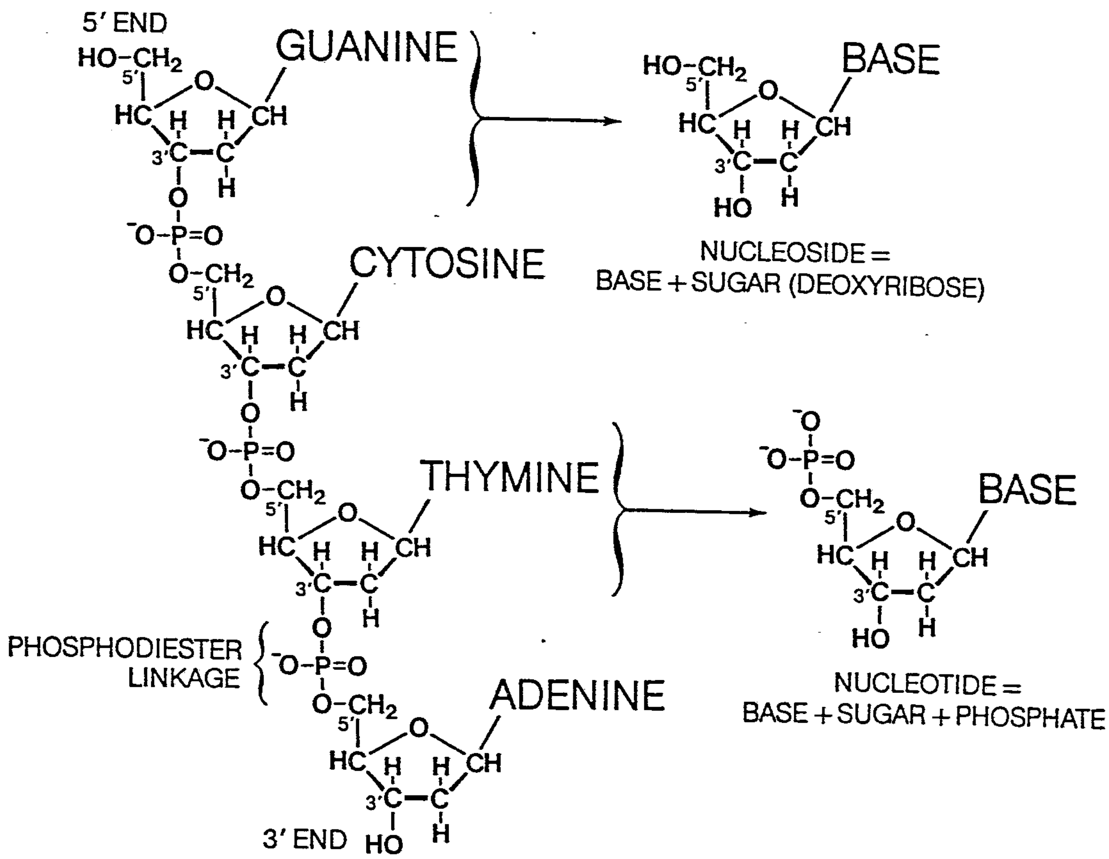


Whereas the phosphodiester bridges and deoxyribose sugar molecule forms a uniform "backbone" throughout the length of the DNA molecule, the sequence of the nucleic acid bases is highly variable. It is this variability of bases along the molecule which specifies a sequence of amino acids (each group of these nucleic bases specifying the cell to produce a single amino acid) in protein. The correspondence between the sequence of nucleic bases in the DNA and the sequence of amino acids in a protein is the basis of the genetic code.

It is now possible to synthesize DNA in the laboratory by adding one nucleotide at a time to an evergrowing nucleotide chain. Such "synthetic DNA" has many important uses in genetic engineering; perhaps the most common use is in the making of "probes" that are used to locate a naturally occurring gene of scientific or commercial interest. Once the specifically-sought gene is located, it may then be characterized, studied, and if desired, duplicated in the laboratory.

The sequencing of oligonucleotides, and particularly DNA, is a valuable tool in understanding the structure and function of the DNA molecule. Present-day sequencing of DNA is conventionally accomplished by one of two methods.

The earlier method for sequencing of DNA was reported by F.S. Sanger, I. Nicklen, and A.R. Coulson (Proceedings of the National Academy of Sciences, 74:5463) in 1977, and requires subcloning of the DNA fragment containing the desired nucleic bases for sequencing into a M13 bacteriophage vector, isolating a single-stranded template DNA, and carrying out the primer extension in the presence of dideoxy chain terminators. Even though the dideoxy chain termination method is relatively simple to perform, the subcloning of the DNA fragment is often very time consuming, and because of this alternative methods were eagerly sought.

The method presently preferred for the sequencing of DNA, is that reported by A. M. Maxam and W. Gilbert (Proceedings of the National Academy of Sciences, 76:560, and Methods in Enzymology, 65:499 (1980) in 1977, the disclosures of which are incorporated herein. Sequencing end-labelled DNA by this method requires the use of four base-specific

reactions as summarized below. In this summary, the procedure for the Guanine & Adenine reaction continues, with the addition of 100 1 of 0.1M piperidine, as for all four base-specific reactions.

| <u>Guanine</u> | <u>Thymine & Cytosine</u> | <u>Cytosine</u> |
|---|---|---|
| 200µl DMS Buffer<br>5µl [$P^{32}$] DNA<br>1µl DMS | 10µl $H_2O_2$<br>10µl [$P^{32}$] DNA<br>30µl hydrazine | 15µl 5M NaCl<br>5µl [$P^{32}$]DNA<br>30µl hydrazine |
| ↓ | ↓ | ↓ |
| 20°C; 10±5 min | 20°C; 10±5min | 20°C; 10±4 min |
| ↓ | ↓ | ↓ |
| 50µl DMS stop | 20µl hydrazine stop | 200µl hydrazine stop |

↓

750µl ethanol

↓

Chill for 5 minutes

↓

Centrifuge

↓

Pellet

↓

250µl 0.3M sodium acetate

↓

750µl ethanol

↓

Chill for 5 minutes

↓

Centrifuge

↓

Pellet

↓

Ethanol Rinse

↓

Vacuum Dry
↓
100 μl 1.0M piperidine
↓
90°C; 30 minutes
↓
lyophilize
↓
10 μl $H_2O$
↓
lyophilize
↓
10 μl $H_2O$
↓
lyophilize
↓
10 μl formamide - NaOH-dyes
↓
90°C; 1 minute
↓
Quick chill
↓
load on gel

<u>Guanine and Adenine</u>

10 μl $H_2O$

10 μl [$P^{32}$] DNA

150 μl formic acid
↓
90°C; 10 minutes
↓
freeze
↓
lyophilize
↓
20 μl $H_2O$
↓
lyophilize

Even though the Maxam-Gilbert procedure is simpler, in that it does not require subcloning of the DNA fragment, the successive steps of (1) base specific modification, (2) stopping the reaction, (3) a series of ethanol precipitations, (4) centrifugations, (5) piperidine cleavage, (6) repeated evaporation and (7) analysis of the cleavage products by gel electrophoresis, evaporation under reduced pressure, are very time consuming. In addition, only small amounts of DNA are normally available, and the potential to lose the DNA pellet during one of the repeated ethanol precipitation or centrifugation steps is high. Furthermore, DNA can co-precipitate with hydrazine during the ethanol precipitation and interferes with the specificity of the reactions. Finally, the piperidine removal usually requires several hours of repeated lyophilizations.

Thus, there have been numerous attempts, prior to the making of the present invention, to overcome the deficiencies of these two methods for the sequencing of DNA fragments. Specifically, a number of attempts have been made to conduct the Maxam-Gilbert reactions on solid supports, a modification which many believed should eliminate the DNA losses, and substantially reduce the time necessary to prepare the DNA sample for placement on the gel. Chuvpilo and Kravchenko (Biolog. Khim. 9:1694) reported in 1980 upon a modification utilizing DE81 anion exchange paper for binding various oligonucleotides before carrying out the Maxam-Gilbert reactions. Although the binding took place, the mechanical instability of the paper support was sufficient to make its use less likely to be a method of choice. Recently Rosenthal et al reported (in Nucleic Acid Research 13:1173)

overcoming the instability problem by the use of a new cellulose based carrier medium with anion exchange properties. While using this support these investigators have successfully sequenced oligonucleotides, however, their method is not applicable to longer DNA fragments. Furthermore, the piperidine used to elute the bound DNA from the carrier medium must still be removed from the system by many repeated lyophilizations, a time consuming process.

Thus, alternative procedures are still needed to overcome the deficiencies of the present methods used for the sequencing of oligonucleotides such as DNA.

It is, therefore, an object of the present invention to define a modified Maxam-Gilbert protocol, using solid support carriers for oligonucleotides, such as DNA.

It is a further object of the present invention to provide a description for the automation of sequencing of oligonucleotides, such as DNA.

A still further object of the present invention is to describe a method for the sequencing of oligonucleotides, such as DNA, in which the loss of DNA at any one step in the method is minimized.

A still further object of the present invention is to describe a method for the sequencing of oligonucleotides, such as DNA, which does not require the centrifugation and evaporation steps of the Maxam-Gilbert protocol.

And it is still a further object of the present invention to describe a method for the sequencing of oligonucleotides, such as DNA, which does not require the precipitation of hydrazine with ethanol.

These and further objects of the present invention will become more apparent in the following

description of the present invention and drawings in which: Fig. 1 is a generalized schematic of an automated apparatus for conducting the modified Maxam-Gilbert according to the present invention.

The protocol for the modified Maxam-Gilbert method to perform oligonucleotide, specifically DNA, sequencing according to the present invention may be summarized as follows:

DNA and Reagent

↓

Adsorption on Reverse Phase Chromatography Column

↓

Selective Elution of DNA Complex

↓

Heating at 90°C in the Presence of Piperidine

↓

Adsorption of Reaction Mixture on Reverse Phase Chromatography Column

↓

Selective Elution of DNA Fragments

Analysis by Gel Electrophoresis

The basis from which the present invention evolved was the belief that if DNA (or other oligonucleotide) could be immobilized on an inert and mechanically stable carrier medium, and if the Maxam-Gilbert reactions could be shown to occur using an immobilized DNA, then such a method for sequencing DNA would not only be extremely easier and less time consuming, but it might be possible to provide for the automation of the entire reaction protocol.

The recent popularity of reverse phase high performance liquid chromatography as an analytical technique in biochemical research can be attributed to the introduction of microparticulate reverse-phase packings in which the packing, such as silica, is reacted with an organic material to chemically carry an aliphatic group. One such carrier material is packaged into columns and sold by Waters Associates, a division of Millipore Corporation, under the designation of $C_{18}$ reverse phase columns. Recently Khorana and his colleagues reported (Proc. Nat. Acad. Sci. USA 81:2285) purification of oligonucleotides, as well as DNA fragments, of up to 1.6 kb on $C_{18}$ reverse-phase columns. Using this method, I have observed that fragments up to 25 kb can be quantitatively (in excess of 90%) eluted from these $C_{18}$ reverse phase columns. Although neither the principles of DNA binding to these columns nor the retention mechanism on these columns is clearly understood, it is believed that the binding does not involve covalent interactions. Since non-covalent interactions are involved in the binding of DNA to these columns, the present invention anticipated such bound DNA would be available for chemical modification reactions.

Using $C_{18}$ reverse phase columns, although other functionally equivalent solid supports may be substituted for the $C_{18}$ material, the present invention has led to a considerably simplified modification of the Maxam-Gilbert method for the sequencing of DNA. Subsequently, the modified method led to the development of an automated system for conducting the sequencing of DNA. Once it was determined DNA would bind to the column, the modified method was developed

as a result of the following seven properties unknown prior to the making of the present invention:

1. Dimethyl Sulfate (DMS), formic acid (HCOOH) and hydrazine ($NH_2$-$NH_2$) react with immobilized DNA on the $C_{18}$ support in a fashion similar to that in solution;
2. Formic acid elutes approximately 20% of the DNA from the $C_{18}$ support;
3. $NH_2$-$NH_2$ does not elute the DNA;
4. Piperidine elutes the DNA.
5. Piperidine upon neutralization to piperidine acetate facilitates DNA binding but does not elute the bound DNA;
6. Thirty-percent (v/v) aqueous acetonitrile elutes the DNA; and
7. Five-percent (v/v) acetonitrile in 25mM triethylammonium bicarbonate (TEAC) washes out $NH_2$-$NH_2$ (hydrazine), HCOOH (formic acid), and DMS, but does not elute the bound DNA.

The method for the sequencing of DNA according to the present invention utilizes the conventional reagents used in the Maxam-Gilbert protocol. Specifically, these are the following:

DMS Buffer
50mM sodium cacodylate (pH 8.0)
1mM EDTA

DMS Stop Solution
1 M Tris-acetate (pH 7.5)
1 M Mercaptoethanol
1.5 M NaOAc
0.5 M $Mg(OAc)_2$
1mM EDTA
0.1mg/ml tRNA

Hydrazine Stop Solution
0.3 M NaOAc
0.01 M $Mg(OAc)_2$
0.1mM EDTA
25μg/ml tRNA

Loading dye
20% Formamide, deionized
50mM Tris-borate (pH 8.3)
1mM EDTA
0.1%(w/v) Xylene Cyanol
0.1%(w/v) Bromophenol Blue

Once the aforementioned seven properties were determined, a process was developed, initially based upon the Maxam-Gilbert sequencing protocol, for the sequencing of oligonucleotides, specifically DNA. A more complete understanding of the sequencing protocol according to the present invention may be had by reference to the following example and description of the accompanying figure.

EXAMPLE I

Four $C_{18}$ Sep-pak reverse phase silica gel chromatographic columns (available from Waters Associates, a division of Millipore Corporation) were labelled G, A, T, and C. These were mounted into four erlenmayer filtering flasks, and the flasks were connected to a vacuum source. Each column was stabilized by passing (with the aid of vacuum) a rinsing solution of 1ml of water and 1 ml of TEAC through the column. An aqueous solution of end labelled (with $P^{32}$) DNA was then passed through the column. The DNA now-immobilized on the $C_{18}$ cartridge, was treated with premixed base specific solutions, according to the Maxam-Gilbert protocol, by soaking the columns with these solutions. Specifically, dimethyl sulfate solution was soaked through and kept for 2 minutes in the G cartridge; similarly 50% formic acid was soaked through and kept in the A cartridge for 4 minutes; 60% hydrazine was soaked through and kept in the T cartridge for 3 minutes; and 60% hydrazine containing 1.5M NaCl was soaked through and kept in the C cartridge for 4 minutes. Stop solutions for G, A, T and C, as given in the Maxam-Gilbert protocol, were passed through the respective columns. Each of the columns were then washed with 5% acetonitrile in 25mM TEAC (20 ml), and the DNA subsequently eluted off of the columns with 200 1 of 10% piperidine. The eluants were heated at 90°C for 30 minutes, followed by the addition of an equal volume of 1N acetic acid to each volume of eluant to form piperidine acetate.

A second set of four fresh $C_{18}$ cartridges were preequilibrated with water and TEAC as before, and the piperidine acetate-containing solution was passed through the column, the columns were washed with 5% acetonitrile in 25mM TEAC, and the final product containing the appropriately cleaved DNA fragments was eluted from the column with 0.5 ml of 30% aqueous acetonitrile. The sample was dried for 30 minutes in a speedvac, 15 to 20$\mu$l of the loading dye was added, and the sample was ready for loading on conventional sequencing gels.

When compared with the conventional Maxam-Gilbert protocol, the protocol outlined in the example offers several advantages. For example, the problem with the concurrent precipitation of hydrazine and ethanol is eliminated; the tedious and time consuming ethanol precipitations and centrifugation steps are eliminated; there is essentially no loss of the DNA sample during the procedure; and the removal of piperidine as the acetate disperses with the need of repeated evaporations under reduced pressure. Most importantly, with the improved method according to the present invention the sequencing procedure can now be automated.

Figure 1 shows a generalized schematic of an automated device for conducting the sequencing of oligonucleotides, specifically DNA, according to the present invention. Although the figure describes a specific configuration for such a device, obviously modifications may be made to the depicted apparatus, and such modifications are considered to be within the scope of the present invention.

The various components of the present invention include a pressure guage PG (this is in the line from the air supply means used to pump the sample and reagent materials through the reverse phase columns; obviously alternatives to an air pump may also be used to achieve the same results); various reagent reservoirs R to hold and disperse the reagents to the reverse phase columns as called for by the automated control means (not shown) utilized to program the device to sequentially run the protocol; valving means V; a raffinate part W for disposal of the spent materials; a fraction collection means FC; and reaction vessels RV. The reverse phase columns for each base specific reaction are shown as G1 and G2

for Guanine, A1 and A2 for Adenine, T1 and T2 for Thymine, and C1 and C2 for Cytosine. Once mounted, the columns are washed with water contained in R1 followed by 25mM TEAC contained in R2. The Maxam-Gilbert reaction products can then be loaded on the columns G1, A1, T1, and C1 either manually or by a series of pumping means (not shown). The columns are then washed with 25mM TEAC contained in R2, followed by 5% acefonitrile in 25mM TEAC contained in R3. The washings are all routed to raffinate port W by appropriate valving means V5 to V8, respectively. The columns are eluted with 1M piperidine contained in R4, and collected in jacketed or heated reaction vessels RV. Once heated at 90°C for 30 min, an equal volume of 1M acetic acid contained in R6 is added to neutralize the piperidine. The neutralized solutions are loaded on to another set of preequilibrated columns G2, A2, T2, and C2, respectively. The washings are repeated and the products eluted with 30% aqueous acetonitrile contained in R5 and collected on a fraction collector FC. The samples can then be dried and run on a sequencing gel.

From the foregoing description, one skilled in the art can easily ascertain the essential characteristics of my invention and without departing from the spirit and scope thereof, can make various changes and/or modifications to the invention for adapting it to various usages and conditions. Accordingly, such changes and modifications are properly intended to be within the full range of equivalents of the following claims.

I CLAIM:

1. A method for the sequencing of oligonucleotides which comprises immobilizing the oligonucleotide to be sequenced onto a solid support carrier material, and adding Maxam-Gilbert base specific solutions to the immobilized oligonucleotide.

2. A method for the sequencing of oligonucleotide which comprises:

- adding Maxam-Gilbert base specific solutions to the oligonucleotide to be sequenced,
- adsorbing the resultant material onto a reverse phase chromatographic solid support material,
- selectively eluting the DNA complex from said support material,
- heating said eluted material in the presence of piperidine,
- adsorbing the reaction products obtained upon heating on reverse phase chromatographic solid support material,
- selectively eluting the oligonucleotide fragments, and
- analyzing the oligonucleotide sequence of said fragment.

3. An apparatus for conducting the sequencing of oligonucleotides of Claim 1.

1/1

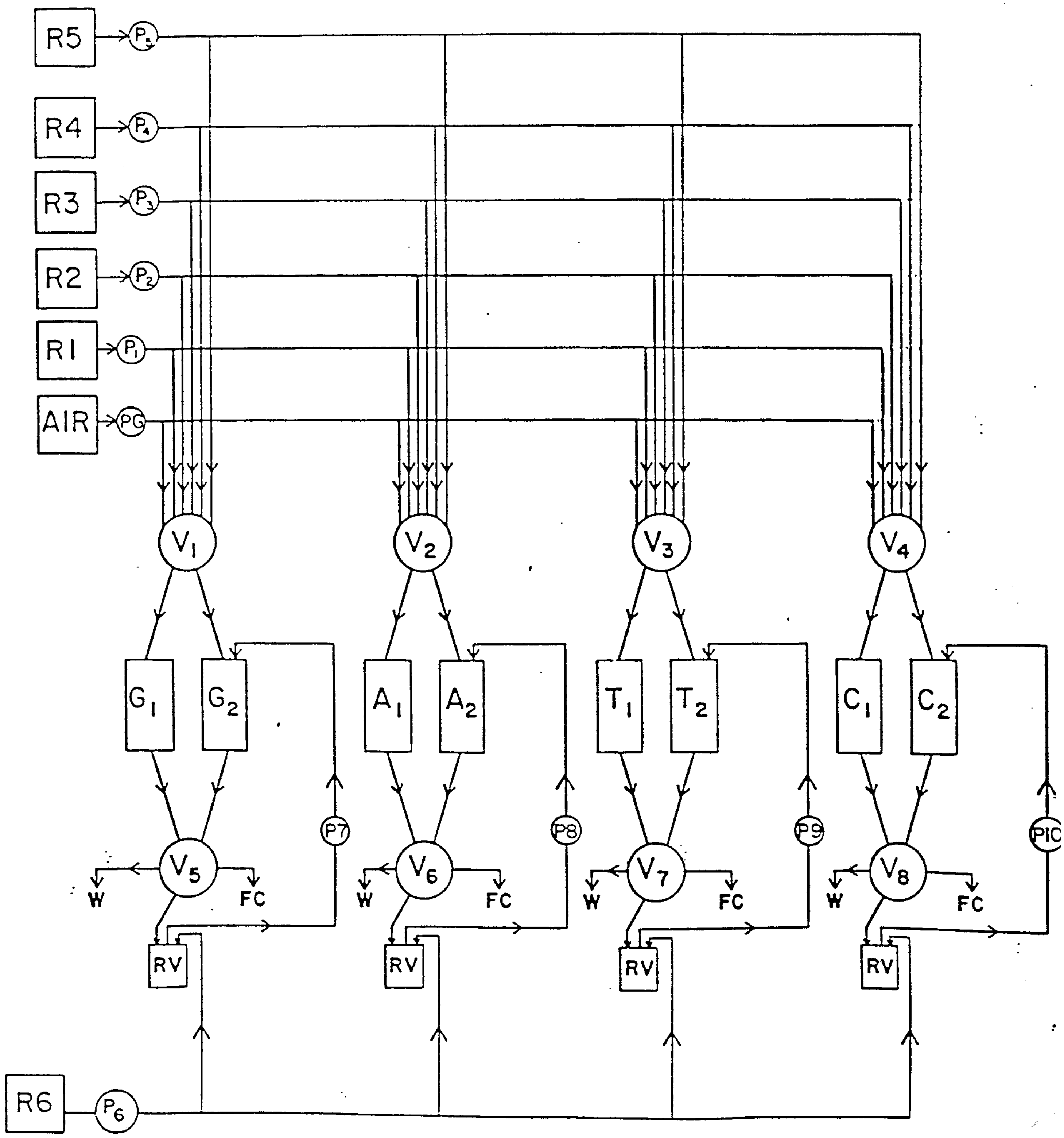
R5
$P_5$
R4
$P_4$
R3
$P_3$
R2
$P_2$
R1
$P_1$
AIR
PG
$V_1$
$V_2$
$V_3$
$V_4$
$G_1$
$G_2$
$A_1$
$A_2$
$T_1$
$T_2$
$C_1$
$C_2$
P7
P8
P9
P10
$V_5$
$V_6$
$V_7$
$V_8$
W
FC
RV
R6
$P_6$